# FASCICULE DE BREVET EUROPEEN

(11) **EP 2 176 052 B1**
(45) Date de publication et mention de la délivrance du brevet: **15.01.2014**
(21) Numéro de dépôt: 08831757.3
(22) Date de dépôt: 18.07.2008
(51) Int. Cl.: B29C 49/42, A61L 2/06, B29C 49/46, B29C 49/06

(54) **PROCÉDÉ ET INSTALLATION DE FABRICATION DE RÈCIPIENTS À PARTIR DE PRÉFORMES EN MATIÈRE THERMOPLASTIQUE**
VERFAHREN SOWIE EINRICHTUNG ZUR HERSTELLUNG VON BEHÄLTERN AUS THERMOPLASTISCHEN VORFORMLINGEN
METHOD AND ISTALLATION FOR MAKING CONTAINERS FROM THERMOPLASTIC PREFORMES

(30) Priorité: 27.07.2007 FR 0705516
(43) Date de publication de la demande: 21.04.2010
(73) Titulaire: Sidel Participations, 76930 Octeville Sur Mer (FR)
(72) Inventeur: FEUILLOLEY, Guy, F-76930 Octeville sur Mer (FR); PLANTAMURA, Bernard, F-76930 Octeville sur Mer (FR)
(74) Mandataire: Demulsant, Xavier
(86) Numéro de dépôt international: PCT/FR2008/001060
(87) Numéro de publication internationale: WO 2009/037396

(56) Documents cités:
- WO-A-98/06559
- DE-A1- 10 118 242
- DE-A1- 19 602 522
- DE-A1-102004 054 938
- GB-A- 943 150

## Description

L'invention se rapporte à la fabrication des récipients à partir de préformes en matière plastique telle que PET (polyéthylène téréphtalate).

Les principales étapes de la fabrication des récipients par étirage soufflage sont les suivantes. Chaque préforme, issue d'une trémie, est d'abord chauffée au défilé dans un four, à une température supérieure à la température de transition vitreuse de sa matière constitutive (pour le PET, cette température est d'environ 70°C). Au sortir du four, la préforme est ensuite introduite chaude dans un moule dont la paroi définit la forme du récipient final. La préforme est alors étirée au moyen d'une tige d'élongation, tandis que de l'air y est introduit sous pression (d'abord aux environs de 10 bar, puis aux environs de 40 bar) pour plaquer la matière, ramollie par le chauffage, contre la paroi du moule. Le récipient ainsi formé est ensuite évacué du moule pour être soit stocké, soit directement rempli puis bouché.

A plusieurs reprises, voire en permanence, le récipient est, au cours de sa fabrication, en contact avec l'air libre (atmosphérique). Or il est connu que l'air atmosphérique est un vecteur de contamination microbiologique. Dans ces conditions, le récipient final a de fortes chances d'être porteur de microbes. Le récipient étant ensuite rempli de liquide, il existe un risque - certes infime mais dont on doit toutefois se prémunir - que le consommateur ingère des agents pathogènes.

Diverses solutions ont été proposées par le passé pour diminuer les risques de contamination des récipients.

Une première technique - actuellement la plus répandue sur le marché - consiste à traiter les récipients par la voie chimique, en aspergeant leur paroi interne d'une solution antiseptique, généralement à base d'acide peracétique ou de peroxyde d'hydrogène (H₂O₂).

Cette première technique ne va pas sans inconvénients.

D'abord, elle requiert une importante consommation d'eau stérile (utilisée pour le rinçage).

Ensuite, cette technique produit des résidus polluants (notamment S001 B028 EP - TQD l'eau de rinçage) qui, soit sont dispersés dans l'environnement, soit - en fonction des normes de pollution en vigueur - doivent subir un dépollution impliquant de complexes et coûteuses mesures de récupération puis de traitement des rejets.

Une seconde technique, connue du brevet américain US 6 315 939, consiste à mettre à profit le soufflage du récipient pour y introduire un mélange gazeux explosif dont l'allumage produit une explosion accompagnée d'une libération d'énergie calorifique suffisante pour stériliser la paroi interne du récipient ainsi traité (en théorie du moins, car à ce jour cette technique ne semble pas avoir été mise en oeuvre à l'échelle industrielle).

On pourra également se référer au document WO 98/06559 (CREATEC)

Cette seconde technique semble séduisante mais, compte tenu du nombre important de récipients à traiter (plusieurs dizaines de milliers par heure), elle met en jeu des volumes considérables de gaz combustible, avec des conséquences néfastes en termes de coût et de sécurisation des installations.

L'invention vise notamment à remédier aux inconvénients précités des techniques connues, en proposant une nouvelle technique de décontamination qui soit efficace tout en étant assez simple à mettre en oeuvre, mais également économique et générant peu (voir pas du tout) de résidus polluants dans le récipient.

A cet effet, l'invention propose, suivant un premier aspect, un procédé de fabrication de récipients à partir de préformes en matière thermoplastique, selon l'une des revendications 1, 2 ou 3.

Suivant un deuxième aspect, l'invention propose une installation de fabrication de récipents à partir de préformes en matière thermoplastique selon la revendication 15.

D'autres objets et avantages de l'invention apparaîtront à la lumière de la description faite ci-après en référence aux dessins annexés dans lesquels :
- la figure 1 est une vue schématique partielle d'une installation de fabrication de récipients par étirage soufflage à partir de préformes en matière thermoplastique, qui comprend un dispositif de traitement des préformes selon l'invention suivant un premier mode de mise en oeuvre (incluant deux variantes) ;
- la figure 2 est une vue schématique partielle d'une installation de fabrication de récipients par étirage soufflage à partir de préformes en matière thermoplastique, qui comprend un dispositif de traitement des préformes selon l'invention suivant une seconde mise en oeuvre ;
- les figures 3A à 3F montrent différentes opérations successives d'un procédé de traitement de préformes conformément à une première configuration de l'invention ;
- les figures 4A à 4E montrent différentes opérations successives d'un procédé de traitement de préformes conformément à une deuxième configuration de l'invention ;
- les figures 5A à 5E montrent différentes opérations successives d'un procédé de traitement de préformes conformément à une troisième configuration de l'invention ;

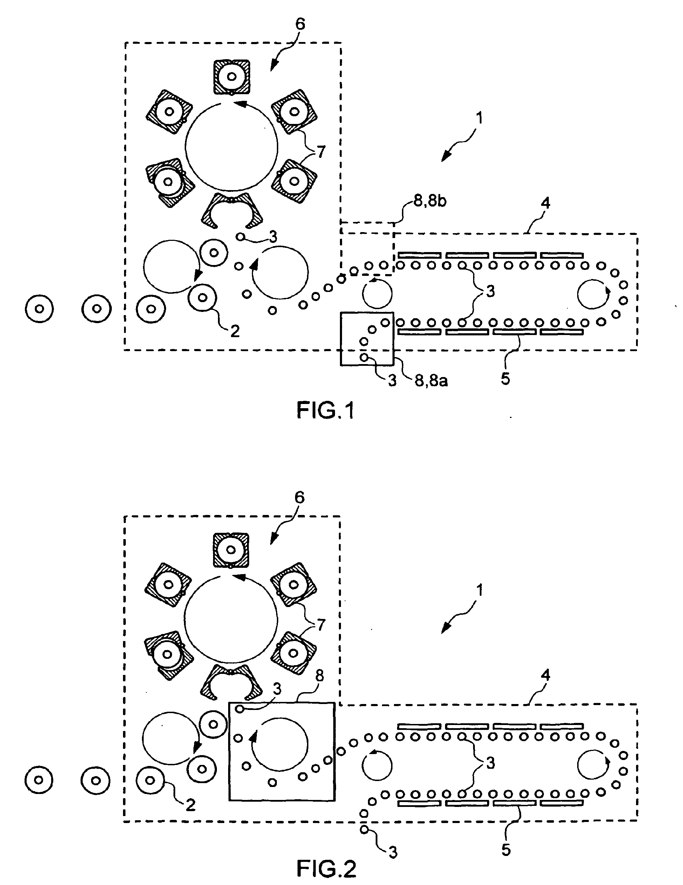

Sur la figure 1 est représentée une installation **1** pour la fabrication de récipients **2** à partir de préformes **3** en matière thermoplastique telle que PET.

Cette installation **1** comprend, en premier lieu, une unité **4** de chauffe ou four dans lequel les préformes **3**, acheminées par une unité d'alimentation en provenance d'une trémie (non représentée), sont chauffées à une température supérieure à la température de transition vitreuse de leur matière constitutive, en défilant devant une succession de lampes **5** à infrarouge.

L'installation **1** comprend, en second lieu, une unité **6** de moulage comprenant une pluralité de moules **7** montés sur un carrousel et dans lesquels, au sortir du four **4**, les préformes **3** sont introduites chaudes en vue d'être étirées et soufflées pour former les récipients **2** finaux.

Préalablement à son introduction dans un moule **7**, chaque préforme **3** est soumise à un traitement de décontamination qui comprend une opération d'introduction dans la préforme **3** de gaz dont le mélange est explosif, suivie d'une opération d'allumage du mélange.

L'installation **1** comprend à cet effet une unité **8** de traitement des préformes **3**, qui comprend une source **9** de gaz carburant tel que de l'hydrogène (H₂), du monoxyde de carbone (CO) ou de l'acétylène (C₂H₂), et éventuellement une source **10** de gaz comburant tel que de l'oxygène (O₂). Ces gaz carburant et comburant peuvent être injectés à l'état pur, ou encore mélangés à des gaz inertes ou réactifs (air, azote, etc.). Le gaz carburant peut être un hydrocarbure issu d'une phase liquide (c'est notamment le cas pour l'acétylène).

Ces sources **9**, **10** peuvent être des bombonnes interchangeables intégrées à l'installation **1**, ou des réservoirs de grande capacité disposés en dehors de l'installation **1**. On peut également prévoir de produire les gaz réactifs en ligne. L'unité **8** de traitement comprend en outre un système **11** d'injection du (ou des) gaz dans les préformes **3**, auquel les sources **9**, **10** sont reliées par des canalisations **12**, ainsi qu'un système **13** d'allumage du mélange, telle qu'une bougie dont l'alimentation en courant génère entre deux électrodes une étincelle suffisante pour provoquer l'allumage et l'explosion du mélange.

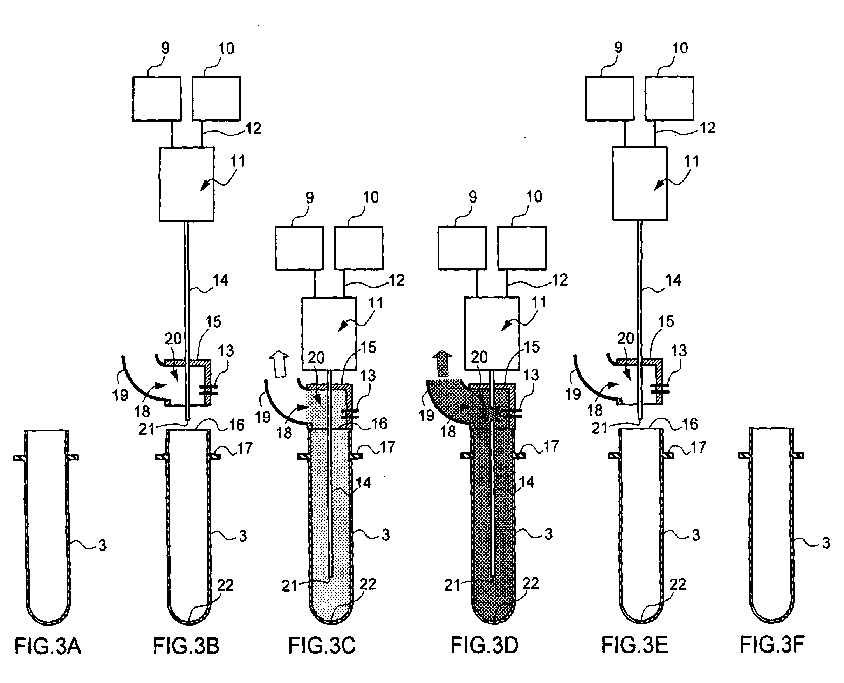

Dans l'hypothèse où l'unité **8** de traitement comprend plusieurs sources **9**, **10** de gaz, leur mélange, dont les proportions (par exemple stoechiométriques) sont prédéterminées pour le rendre explosif, peut être réalisé soit au sein du système **11** d'injection (injection indirecte), soit dans la préforme **3** elle-même (injection directe). Dans ce second cas, illustré sur les figures 3 à 5, les canalisations **12** d'amenée des gaz au système **11** d'injection convergent, le mélange étant introduit dans chaque préforme **3** par une canule **14**.

Suivant un premier mode de réalisation, illustré sur la figure 1, l'unité **8** de traitement est installée en amont du four **4**. Injection des gaz dans chaque préforme **3** et allumage du mélange sont réalisés avant la chauffe.

En variante, sur cette même figure, l'unité **8** de traitement est séparée en deux modules : un module **8a** d'injection du ou des gaz, disposé en amont du four **4**, et un module **8b** d'allumage du mélange, disposé en aval du four **4**. Dans cette dernière configuration, les gaz sont injectés dans chaque préforme **3** avant sa chauffe, l'allumage étant effectué après celle-ci. Il est à noter que cette configuration suppose que soient prévus des moyens de bouchage des préformes **3** pendant la chauffe.

Suivant un deuxième mode de réalisation, illustré sur la figure 2, l'unité **8** de traitement est intercalée entre le four **4** et l'unité **6** de moulage, l'introduction du mélange gazeux explosif et son allumage étant tous deux réalisés à l'issue de l'opération de chauffe.

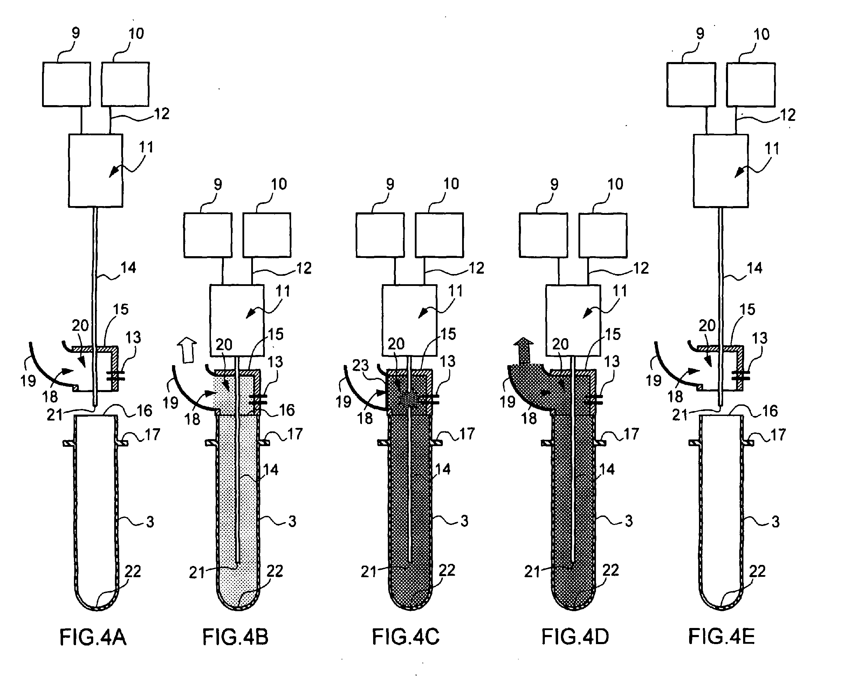

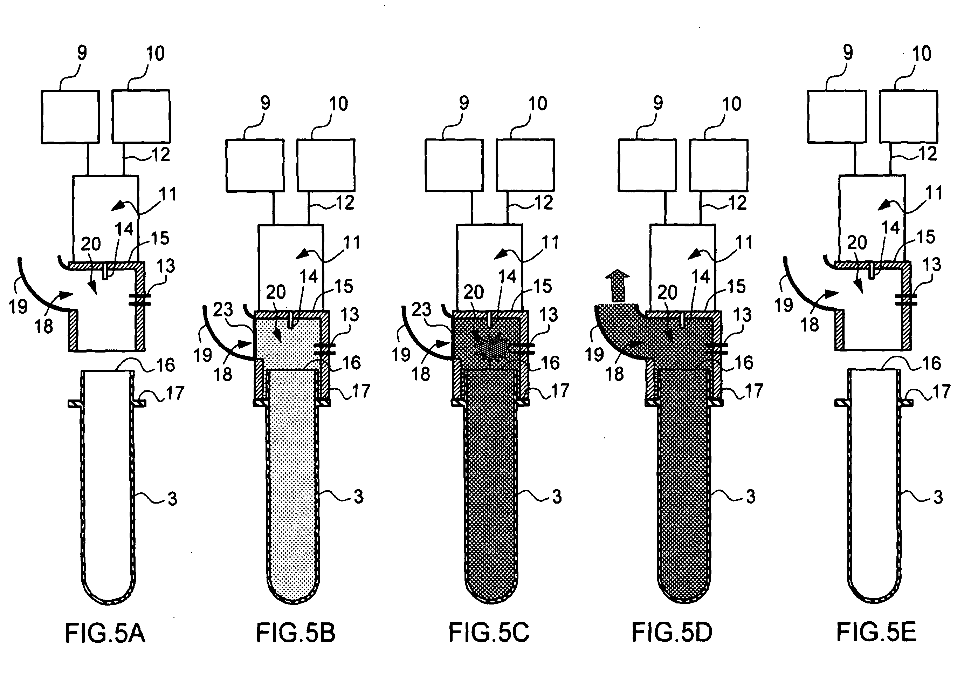

Comme cela est visible sur les figures 3 à 5, l'unité **8** de traitement est pourvue d'une culasse **15** mobile entre une position basse dans laquelle il est appliqué de manière étanche sur un col **16** (figures 2 et 4) ou une collerette **17** (figure 5) de chaque préforme **3**, et une position haute dans laquelle il est écarté de la préforme **3**. Cette culasse **15** comprend une ouverture **18** latérale, éventuellement prolongée par une tubulure **19**, pour l'échappement au moins partiel des gaz présents dans la préforme **3**, que ce soit avant ou à l'issue de la combustion.

Suivant un mode de réalisation illustré sur les figures 3 à 5, la culasse **15** définit une chambre **20** de combustion qui, en position basse, surplombe la préforme **3** et dans laquelle débouchent les électrodes de la bougie **13**.

Au moins trois configurations peuvent être envisagées, selon les conditions d'injection et de pression du mélange gazeux.

Dans une première configuration, illustrée sur la figure 3 (figures 3A à 3F), une opération de balayage de la préforme **3** est prévue au moyen des gaz introduits dans celle-ci, afin de substituer le mélange explosif à l'air initialement présent dans la préforme **3**. Par ailleurs, la préforme **3** est maintenue à l'air libre pendant qu'est réalisé l'allumage du mélange gazeux explosif.

Plus précisément, dans cette configuration, 1 le procédé de décontamination comprend une première étape d'introduction de la préforme **3** dans l'unité **8** de traitement (figure 3A), suivie d'une deuxième étape de descente de la culasse **15** qui vient s'appliquer sur le buvant du col **16** de la préforme **3** (figure 3B). Comme nous le verrons ci-après, la culasse **15** peut être appliquée sur une autre partie du récipient, telle que sa collerette **17**.

Une étape suivante comprend la descente de la canule **14** et/ou de l'ensemble du système **11** d'injection (comme représenté sur la figure 3C) de sorte qu'une extrémité **21** inférieure de la canule **14**, par laquelle le mélange gazeux est introduit dans la préforme **3**, se trouve à proximité du fond **22** de la préforme **3**.

Une étape suivante (figure 3C) consiste à effectuer un balayage de la préforme **3** au moyen du mélange gazeux explosif, lequel, injecté dans la préforme **3** par la canule **14** à la pression atmosphérique, se substitue à l'air initialement présent qui est évacué par l'ouverture **18** dans la culasse **15**, comme l'indique la flèche sur la figure 3C.

Il est à noter que la préforme **3** peut être balayée à l'air libre sans descente de la culasse **15**, ou tout du moins sans que celle-ci ne soit appliquée contre le buvant du col **16** de la préforme **3**. Dans une telle configuration, le dispositif d'allumage pourra être monté directement sur la canule **14**.

La préforme **3** étant maintenue à l'air libre (l'ouverture **18** dans la culasse **15** n'étant pas fermée), une étape suivante (figure 3D) consiste à produire dans la chambre **20** de combustion une étincelle au moyen de la bougie **13** de sorte à allumer le mélange gazeux et provoquer ainsi sa combustion explosive. Cette explosion est accompagnée dans la préforme **3** d'un dégagement momentané de chaleur (pic thermique), sans toutefois que l'on note une augmentation substantielle de la pression (pic de pression) du fait du maintien de la préforme **3** à l'air libre. Les gaz issus de la combustion s'échappent par l'ouverture **18** dans la culasse **15** puis par la tubulure **19**, comme l'indique la flèche sur la figure 3D.

Une fois la combustion achevée, le système **11** d'injection et la culasse **15** sont soulevés (figure 3E) et la préforme **3** évacuée de l'unité **8** de traitement.

Des simulations ont été effectuées en application de ce procédé sur une préforme en PET de 23 g pour la fabrication d'une bouteille de 340 ml (volume interne de la préforme : 20,7 ml ; surface interne : 42,8 cm²). Deux types de mélanges gazeux explosifs ont été employés : un mélange hydrogène-oxygène, et un mélange monoxyde de carbone-oxygène, dans des proportions stoechiométriques.

Les paramètres de la simulation et leurs valeurs respectives sont listés dans le tableau ci-dessous.

| Mélange | H₂ + ½ O₂ | CO + ½ O₂ |
|---|---|---|
| Pression du mélange (bar) | 1 | 1 |
| Energie dégagée (J) | 137 | 161 |
| Température des gaz (°C) | 6950 | 7500 |
| Énergie surfacique (J/cm²) | 3,2 | 3,8 |
| Pression maximale (bar) | 1 | 1 |
| Échauffement de la préforme (°C) | 2 | 2 |

Dans une deuxième configuration, illustrée sur la figure **4** (figures 4A à 4E), une opération de balayage de la préforme **3** est prévue au moyen du mélange gazeux explosif, afin de substituer celui-ci à l'air initialement présent dans la préforme **3**. Cependant, la préforme **3** est confinée pendant l'allumage et pendant l'explosion du mélange gazeux, celui-ci étant introduit dans la préforme **3** à la pression atmosphérique.

Après introduction de la préforme **3** dans l'unité **8** de traitement et descente de la culasse **15** qui vient s'appliquer sur le col **16** de la préforme **3** (figure 4A), l'ensemble du système **11** d'injection est descendu (comme représenté sur la figure 4B) de sorte qu'une extrémité inférieure **21** de la canule **14**, par laquelle le mélange gazeux est introduit dans la préforme **3**, se trouve à proximité du fond **22** de la préforme **3**.

Un balayage de la préforme **3** est ensuite effectué au moyen du mélange gazeux explosif, lequel, injecté dans la préforme **3** par la canule **14** à la pression atmosphérique, se substitue à l'air initialement présent qui est évacué par l'ouverture **18** dans la culasse **15**, comme l'indique la flèche sur la figure 4B.

La préforme **3** étant confinée par obturation de l'ouverture **18** pratiquée dans la culasse **15** au moyen d'un volet **23** mobile, une étincelle est produite dans la chambre de combustion au moyen de la bougie **13** de sorte à allumer le mélange gazeux à la pression atmosphérique et provoquer ainsi sa combustion explosive (figure 4C). Cette explosion est accompagnée dans la préforme **3** d'une augmentation momentanée de la pression (pic de pression) du fait du confinement de celle-ci, et d'un dégagement momentané de chaleur (pic thermique).

Le volet **23** est ensuite ouvert pour mettre la préforme **3** à l'air libre et permettre l'échappement des gaz issus de la combustion par l'ouverture **18** dans la culasse **15**, comme l'indique la flèche sur la figure 4D.

Le système **11** d'injection et la culasse **15** sont ensuite soulevés (figure 4E) et la préforme dans la préforme **3** évacuée de l'unité **8** de traitement.

Des simulations ont été effectuées en application de ce procédé sur une préforme en PET de 23 g pour la fabrication d'une bouteille de 340 ml telle que décrite précédemment. Deux types de mélanges gazeux explosifs ont été employés : un mélange hydrogène-oxygène, et un mélange monoxyde de carbone-oxygène, dans des proportions stoechiométriques.

Les paramètres de la simulation et leurs valeurs respectives sont listés dans le tableau ci-dessous.

| Mélange | H₂ + O₂ | CO + O₂ |
|---|---|---|
| Pression du mélange (bar) | 1 | 1 |
| Energie dégagée (J) | 137 | 161 |
| Température du gaz (°C) | 6950 | 7500 |
| Energie surfacique (J/cm²) | 3,2 | 3,8 |
| Pression maximale (bar) | 16,5 | 17,7 |
| Echauffement de la préforme (°C) | 4,8 | 5,6 |
| Echauffement de la surface de la préforme (sur une épaisseur de 0,2 mm) (°C) | 95 | 113 |

Dans une troisième configuration, illustrée sur la figure 5 (figures 5A à 5E), la préforme dans la préforme **3** est confinée pendant les opérations d'injection, d'allumage et d'explosion du mélange gazeux. Celui-ci est introduit dans la préforme **3** à une pression supérieure à la pression atmosphérique, par exemple à environ 3 ou 5 bar (voir les tests décrits ci-après).

La préforme **3** est d'abord introduite dans l'unité **8** de traitement, la culasse **15** et le système **11** d'injection, dont la canule **14** débouche dans la chambre **20** de combustion (figure 5A), étant conjointement descendus jusqu'à la position basse de la culasse **15**, appliqué de manière étanche sur la collerette **17** (mais la culasse pourrait, comme précédemment, être appliquée sur le col **16**).

La préforme **3** étant confinée par obturation de l'ouverture **18** pratiquée dans la culasse **15** au moyen d'un volet **23** mobile, les gaz (mélangés ou non) sont introduits sous pression dans la préforme **3** (figure 5B), puis une étincelle est produite dans la chambre **20** de combustion au moyen de la bougie **13** de sorte à allumer le mélange gazeux sous pression et provoquer ainsi sa combustion explosive (figure 5C). Cette explosion est accompagnée dans la préforme **3** d'une augmentation momentanée de la pression (pic de pression) du fait du confinement de celle-ci, et d'un dégagement momentané de chaleur (pic thermique).

Le volet **23** est ensuite ouvert pour mettre la préforme **3** à l'air libre et permettre l'échappement des gaz issus de la combustion par l'ouverture **18** dans la culasse **15**, comme l'indique la flèche sur la figure 5D.

Le système **11** d'injection et la culasse **15** sont ensuite soulevés (figure 5E) et la préforme **3** évacuée de l'unité **8** de traitement.

Des simulations ont été effectuées en application de ce procédé sur une préforme en PET de 23 g pour la fabrication d'une bouteille de 340 ml telle que décrite précédemment. Deux types de mélanges gazeux explosifs ont été employés : un mélange air-hydrogène et un mélange air-monoxyde de carbone, dans des proportions stoechiométriques. La préforme **3** a été mise en pression avec de l'air, plusieurs pressions ayant été appliquées : 1, 3, et 5 bars.

Les paramètres de la simulation et leurs valeurs respectives sont listés dans le tableau ci-dessous.

| Mélange | Air + H₂ | Air + CO | Air+ H₂ | Air + CO | Air+ H₂ | Air + CO |
|---|---|---|---|---|---|---|
| Pression d'air (absolue, en bar) | 1 | 1 | 3 | 3 | 5 | 5 |
| Quantité d'oxygène dans la préforme (mg) | 5,8 | 5,8 | 17,4 | 17,4 | 28,9 | 28,9 |
| Quantité de gaz carburant injecté (mg) | 0,72 | 10,1 | 2,16 | 30,3 | 3,6 | 50,6 |
| Energie dégagée (J) | 87 | 101 | 259 | 303 | 433 | 506 |
| Température du gaz (°C) | 2700 | 3000 | 2700 | 3000 | 2700 | 3000 |
| Energie surfacique (J/cm²) | 2 | 2,4 | 6,1 | 7,1 | 10,1 | 11,8 |
| Pression maximale (bar) | 12,3 | 13,7 | 36,8 | 41,2 | 61,4 | 68,7 |
| Echauffement de la préforme (°C) | 3,0 | 3,5 | 9,0 | 10,5 | 15,0 | 17,5 |
| Echauffement de la surface de la préforme (sur une épaisseur de 0,2 mm) (°C) | 60 | 71 | 182 | 211 | 301 | 351 |

Deux séries de tests microbiologiques ont été conduits sur un échantillonnage d'une dizaine de préformes issues d'un traitement conforme à la première configuration (balayage et explosion en milieu ouvert), avec respectivement un mélange hydrogène-oxygène et un mélange monoxyde de carbone-oxygène. Les mesures microbiologiques comprennent le nombre de colonies de flore aérobie mésophile, de levure et de moisissure qui se sont développées à l'issue du traitement. Ces mesures ont été comparées à des mesures similaires effectuées sur des préformes non traitées.

Les mesures issues des tests effectués sur les préformes traitées par explosion d'un mélange hydrogène-oxygène sont rassemblés dans le tableau ci-dessous.

| **N° préforme** | **Flore aérobie mésophile** | **Levures** | **Moisissures** |
|---|---|---|---|
| 1 | 0 | 0 | 0 |
| 2 | 0 | 0 | 0 |
| 3 | 0 | 0 | 0 |
| 4 | 0 | 0 | 0 |
| 5 | 2 | 0 | 0 |
| 6 | 1 | 0 | 0 |
| 7 | 3 | 0 | 0 |
| 8 | 4 | 0 | 0 |
| 9 | 1 | 0 | 0 |
| 10 | 0 | 0 | 0 |

Les mesures issues des tests effectués sur les préformes traitées par explosion d'un mélange monoxyde de carbone-oxygène sont rassemblés dans le tableau ci-dessous.

| **N° préforme** | **Flore aérobie mésophile** | **Levures** | **Moisissures** |
|---|---|---|---|
| 1 | 0 | 0 | 0 |
| 2 | 0 | 0 | 0 |
| 3 | 0 | 0 | 0 |
| 4 | 0 | 0 | 0 |
| 5 | 0 | 0 | 0 |
| 6 | 1 | 0 | 1 |
| 7 | 0 | 0 | 0 |
| 8 | 0 | 0 | 0 |
| 9 | 1 | 0 | 0 |
| 10 | 0 | 0 | 0 |

Les mesures issues des tests effectués sur les préformes non traitées rassemblés dans le tableau ci-dessous.

| **N° préforme** | **Flore aérobie mésophile** | **Levures** | **Moisissures** |
|---|---|---|---|
| 1 | 26 | 0 | 22 |
| 2 | 23 | 0 | 20 |
| 3 | 16 | 1 | 16 |
| 4 | 20 | 0 | 20 |
| 5 | 20 | 0 | 12 |
| 6 | 16 | 0 | 18 |
| 7 | 23 | 0 | 19 |
| 8 | 43 | 0 | 27 |
| 9 | 6 | 0 | 6 |
| 10 | 20 | 0 | 14 |

On constate que, même dans les conditions les moins favorables (explosion en milieu ouvert à la pression atmosphérique), la plupart des microbes sont anéantis par le choc thermique accompagnant le traitement subi par les préformes, ce qui témoigne de l'efficacité du procédé proposé.

Dans les conditions les plus favorables (explosion en milieu confiné, éventuellement sous pression), la décontamination est encore améliorée du fait de conditions de température et de pressions plus sévères (choc thermique et souffle).

## Revendications

1. Procédé de fabrication de récipients (**2**) à partir de préformes (**3**) en matière thermoplastique, qui comprend successivement :
- une opération d'introduction dans les préformes (**3**) de gaz dont le mélange est explosif,
- une opération d'allumage du mélange,
- une opération de chauffe des préformes (**3**),
- une opération d'introduction des préformes (**3**) dans un moule (**7**),
- une opération de formage des récipients (**2**) à partir des préformes (**3**).

2. Procédé de fabrication de récipients (**2**) à partir de préformes (**3**) en matière thermoplastique, qui comprend successivement :
- une opération de chauffe des préformes (**3**),
- une opération d'introduction dans les préformes (**3**) de gaz dont le mélange est explosif,
- une opération d'allumage du mélange,
- une opération d'introduction des préformes (**3**) dans un moule,
- une opération de formage des récipients (**2**) à partir des préformes (**3**).

3. Procédé de fabrication de récipients (**2**) à partir de préformes (**3**) en matière thermoplastique, qui comprend successivement :
- une opération d'introduction dans les préformes (**3**) de gaz dont le mélange est explosif,
- une opération de chauffe des préformes (**3**),
- une opération d'allumage du mélange,
- une opération d'introduction des préformes (**3**) dans un moule,
- une opération de formage des récipients (**2**) à partir des préformes (**3**).

4. Procédé selon l'une quelconque des revendications 1 à 3, qui comprend, préalablement à l'allumage, une opération de balayage de la préforme (**3**) au moyen des gaz.

5. Procédé selon l'une quelconque des revendications 1 à 4, dans lequel, lors de l'allumage, la préforme (**3**) est maintenue ouverte à l'air libre.

6. Procédé selon l'une quelconque des revendications 1 à 5, qui comprend, préalablement à l'allumage, une opération de confinement de la préforme (**3**), les gaz étant à la pression atmosphérique.

7. Procédé selon l'une quelconque des revendications 1 à 5, qui comprend, préalablement à l'introduction des gaz, une opération de confinement de la préforme (**3**).

8. Procédé selon l'une quelconque des revendications 1 à 7, dans lequel les gaz sont introduits dans la préforme (**3**) à une pression supérieure à la pression atmosphérique.

9. Procédé selon la revendication 8, dans lequel les gaz sont introduits dans la préforme (**3**) sous une pression d'environ 3 bar.

10. Procédé selon la revendication 8, dans lequel les gaz sont introduits dans la préforme (**3**) à une pression d'environ 5 bar.

11. Procédé selon l'une des revendications 1 à 10, dans lequel les gaz comprennent de l'oxygène et de l'hydrogène.

12. Procédé selon l'une des revendications 1 à 10, dans lequel les gaz comprennent de l'oxygène et du monoxyde de carbone.

13. Procédé selon l'une des revendications 1 à 10, dans lequel l'un au moins des gaz est issu d'un hydrocarbure.

14. Procédé selon l'une des revendications 1 à 10, dans lequel les gaz comprennent de l'oxygène et de l'acétylène.

15. Installation de fabrication de récipients (**2**) à partir de préformes (**3**) en matière plastique, qui comprend :
- une unité (**4**) de chauffe des préformes (**3**),
- une unité (**6**) de moulage comprenant une pluralité de moules (**7**) pour l'introduction des préformes (**3**) chaudes au sortir de l'unité (**4**) de chauffe,
**caractérisée en ce qu'**elle comprend en outre une unité (**8**) de traitement des préformes en amont de l'unité (**6**) de moulage, ladite unité (**8**) de traitement comprenant :
- une ou plusieurs sources (**9**, **10**) de gaz dont le mélange est explosif.
- un système (**11**) d'injection du ou des gaz dans les préformes (**3**),
- des moyens (**13**) d'allumage du mélange dans les préformes.

16. Installation (**1**) selon la revendication 15, qui comprend des moyens (**15**, **23**) de confinement des préformes (**3**).

17. Installation (**1**) selon la revendication 16, dans laquelle les moyens (**15**, **23**) de confinement comprennent une culasse (**15**) mobile susceptible d'être appliquée sur une préforme (**3**).

18. Installation (**1**) selon l'une des revendications 15 à 17, dans laquelle le système (**11**) d'injection comprend une canule (**14**).

19. installation (**1**) selon la revendication 18, dans laquelle la canule (**14**) est montée mobile.

20. Installation (**1**) selon l'une des revendications 15 à 19, dans laquelle l'unité (**8**) de traitement est intercalée entre l'unité (**4**) de chauffe et l'unité (**7**) de moulage.

## Patentansprüche

1. Verfahren zur Herstellung von Behältern (2) aus Vorformlingen (3) aus thermoplastischem Material, nacheinander umfassend:
- einen Schritt der Einleitung von Gasen, deren Gemisch explosiv ist, in die Vorformlinge (3),
- einen Schritt des Zündens des Gemisches,
- einen Schritt des Erhitzens der Vorformlinge (3),
- einen Schritt der Einführung der Vorformlinge (3) in eine Form (7),
- einen Schritt des Formens der Behälter (2) aus Vorformlingen (3).

2. Verfahren zur Herstellung von Behältern (2) aus Vorformlingen (3) aus thermoplastischem Material, nacheinander umfassend:
- einen Schritt der Erhitzung der Vorformlinge (3),
- einen Schritt der Einleitung von Gasen, deren Gemisch explosiv ist, in die Vorformlinge (3),
- einen Schritt des Zündens des Gemisches,
- einen Schritt der Einführung der Vorformlinge (3) in eine Form,
- einen Schritt des Formens von Behältern (2) aus den Vorformlingen (3).

3. Verfahren zur Herstellung von Behältern (2) aus Vorformlingen (3) aus thermoplastischem Material, nacheinander umfassend:
- einen Schritt der Einleitung von Gasen, deren Gemisch explosiv ist, in die Vorformlinge (3),
- einen Schritt der Erhitzung der Vorformlinge (3),
- einen Schritt des Zündens des Gemisches,
- einen Vorgang des Einführens der Vorformlinge (3) in eine Form,
- einen Schritt des Formens der Behälter (2) aus den Vorformlingen (3).

4. Verfahren nach einem der Ansprüche 1 bis 3, das vor der Zündung einen Vorgang des Spülens des Vorformlings (3) mit Hilfe der Gase umfasst.

5. Verfahren nach einem der Ansprüche 1 bis 4, bei dem beim Zünden der Vorformling (3) im Freien offen gehalten wird.

6. Verfahren nach einem der Ansprüche 1 bis 5, das vor dem Zünden einen Schritt der Eingrenzung des Vorformlings (3) umfasst, wobei die Gase unter Atmosphärendruck stehen.

7. Verfahren nach einem der Ansprüche 1 bis 5, das vor der Einleitung der Gase einen Schritt der Eingrenzung des Vorformlings (3) umfasst.

8. Verfahren nach einem der Ansprüche 1 bis 7, bei dem die Gase in den Vorformling (3) mit einem höheren Druck als Atmosphärendruck eingeleitet werden.

9. Verfahren nach Anspruch 8, bei dem die Gase in den Vorformling (3) unter einem Druck von ungefähr 3 bar eingeleitet werden.

10. Verfahren nach Anspruch 8, bei dem die Gase in den Vorformling (3) unter einem Druck von ungefähr 5 bar eingeleitet werden.

11. Verfahren nach einem der Ansprüche 1 bis 10, bei dem die Gase Sauerstoff und Wasserstoff umfassen.

12. Verfahren nach einem der Ansprüche 1 bis 10, bei dem die Gase Sauerstoff und Kohlenmonoxid umfassen.

13. Verfahren nach einem der Ansprüche 1 bis 10, bei dem mindestens eines der Gase aus einem Kohlenwasserstoff stammt.

14. Verfahren nach einem der Ansprüche 1 bis 10, bei dem die Gase Sauerstoff und Acetylen umfassen.

15. Anlage zur Herstellung von Behältern (2) aus Vorformlingen (3) aus Kunststoff, umfassend:
- eine Einheit (4) zum Erhitzen der Vorformlinge (3),
- eine Formungseinheit (6), umfassend eine Vielzahl von Formen (7) für die Einführung der heißen Vorformlinge (3) beim Austritt aus der Erhitzungseinheit (4),
**dadurch gekennzeichnet, dass** sie ferner eine Einheit (8) zur Bearbeitung der Vorformlinge stromaufwärts zu der Formungseinheit (6) umfasst, wobei die Bearbeitungseinheit (8)
- eine oder mehrere Quellen (9, 10) von Gasen, deren Gemisch explosiv ist,
- ein System (11) zum Einspritzen des oder der Gase in die Vorformlinge (3),
- Mittel (13) zum Zünden des Gemisches in den Vorformlingen.
umfasst.

16. Anlage (1) nach Anspruch 15, umfassend Mittel (15, 23) zur Eingrenzung der Vorformlinge (3).

17. Anlage (1) nach Anspruch 16, bei der die Eingrenzungsmittel (15, 23) ein bewegliches Joch (15) umfassen, das auf einen Vorformling (3) gesetzt werden kann.

18. Anlage (1) nach einem der Ansprüche 15 bis 17, bei der das Einspritzsystem (11) eine Kanüle (14) umfasst.

19. Anlage (1) nach Anspruch 18, bei der die Kanüle (14) beweglich montiert ist.

20. Anlage (1) nach einem der Ansprüche 15 bis 19, bei der die Bearbeitungseinheit (8) zwischen der Erhitzungseinheit (4) und der Formungseinheit (7) angeordnet ist.

## Claims

1. Process for manufacturing containers (2) from thermoplastic preforms (3), which comprises in succession:
- an operation of introducing into the preforms (3) gases the mixture of which is explosive;
- an operation of igniting the mixture;
- an operation of heating the preforms (3);
- an operation of introducing the preforms (3) into a mould (7); and
- an operation of forming containers (2) from the preforms (3).

2. Process for manufacturing containers (2) from thermoplastic preforms (3), which comprises in succession:
- an operation of heating the preforms (3);
- an operation of introducing into the preforms (3) gases the mixture of which is explosive;
- an operation of igniting the mixture;
- an operation of introducing the preforms (3) into a mould; and
- an operation of forming containers (2) from the preforms (3).

3. Process for manufacturing containers (2) from thermoplastic preforms (3), which comprises in succession:
- an operation of introducing into the preforms (3) gases the mixture of which is explosive;
- an operation of heating the preforms (3);
- an operation of igniting the mixture;
- an operation of introducing the preforms (3) into a mould; and
- an operation of forming containers (2) from the preforms (3).

4. Process according to any one of Claims 1 to 3, which comprises, before the ignition, an operation of flushing the preform (3) by means of the gases.

5. Process according to any one of Claims 1 to 4, in which, during ignition, the preform (3) is kept open to free air.

6. Process according to any one of Claims 1 to 5, which comprises, before the ignition, an operation of confining the preform (3), the gases being at atmospheric pressure.

7. Process according to any one of Claims 1 to 5, which comprises, before introduction of the gases, an operation of confining the preform (3).

8. Process according to any one of Claims 1 to 7, in which the gases are introduced into the preform (3) at a pressure higher than atmospheric pressure.

9. Process according to Claim 8, in which the gases are introduced into the preform (3) under a pressure of about 3 bar.

10. Process according to Claim 8, in which the gases are introduced into the preform (3) at a pressure of about 5 bar.

11. Process according to one of Claims 1 to 10, in which the gases comprise oxygen and hydrogen.

12. Process according to one of Claims 1 to 10, in which the gases comprise oxygen and carbon monoxide.

13. Process according to one of Claims 1 to 10, in which at least one of the gases is derived from a hydrocarbon.

14. Process according to one of Claims 1 to 10, in which the gases comprise oxygen and acetylene.

15. Plant for manufacturing containers (2) from plastic preforms (3), which comprises:
- a unit (4) for heating the preforms (3); and
- a moulding unit (6) comprising a plurality of moulds (7) for introduction of the hot preforms (3) at the output of the heating unit (4),
**characterised in that** it furthermore comprises a unit (8) for processing the preforms upstream of the moulding unit (6), said processing unit (8) comprising:
- one or more sources (9, 10) of gases the mixture of which is explosive;
- a system (11) for injecting the one or more gases into the preforms (3); and
- means (13) for igniting the mixture in the preforms.

16. Plant (1) according to Claim 15, which comprises means (15, 23) for confining the preforms (3).

17. Plant (1) according to Claim 16, in which the confining means (15, 23) comprising a movable head (15) capable of being applied to a preform (3).

18. Plant (1) according to one of Claims 15 to 17, in which the injecting system (11) comprises a cannula (14).

19. Plant (1) according to Claim 18, in which the cannula (14) is movably mounted.

20. Plant (1) according to one of Claims 15 to 19, in which the processing unit (8) is intermediate between the heating unit (4) and the moulding unit (7).
